# EUROPEAN PATENT SPECIFICATION

(11) **EP 3 243 479 B1**
(45) Date of publication and mention of the grant of the patent: **04.03.2020**
(21) Application number: 15867442.4
(22) Date of filing: 02.12.2015
(51) Int. Cl.: A61C 8/00, A61L 27/36, A61L 2/14, A61C 13/00

(54) **METHOD FOR PRODUCING IMPLANTS WITH A PERSONALISED SURFACE**
VERFAHREN ZUR HERSTELLUNG VON IMPLANTATEN MIT EINER PERSONALISIERTEN OBERFLÄCHE
PROCÉDÉ POUR L'OBTENTION D'IMPLANTS À SURFACE PERSONNALISÉE

(30) Priority: 09.12.2014 ES 201431805
(43) Date of publication of application: 15.11.2017
(73) Proprietor: Fermoinvers, S.L., 47197 Valladolid (ES)
(72) Inventor: MOZO GRAU, Fernando, 47197 Valladolid (ES)
(74) Representative: Pons
(86) International application number: PCT/ES2015/070871
(87) International publication number: WO 2016/092130

(56) References cited:
- WO-A1-00/44314
- WO-A1-02/24243
- US-A- 5 071 351
- US-A1- 2008 292 779
- US-A1- 2010 036 502
- US-A1- 2014 004 481
- US-B1- 6 214 049

## Description

### OBJECT OF THE INVENTION

The invention refers to a procedure for producing implants with a personalised surface, as defined in the appended claims.

In particular, the object of the invention is a procedure, the aim of which is to produce an implant with a surface treated in a personalised manner to cover each need. It essentially consists of a cleaning/activation process carried out with plasma technology to ensure that the implant surface is clean and chemically receptive, and the application of different types of liquid solutions (osteoinductive, osteoconductive, antibiotic, probiotic or antibacterial), based on the specific needs of each patient, with the primary objective of accelerating osseointegration in dental implantology and increasing the BIC (*bone implant contact*) values between the bone and the implant.

### FIELD OF THE INVENTION

The field of this invention is the dentistry field and in particular, the oral implantology area.

### BACKGROUND OF THE INVENTION

As is known, the use of implants to treat tooth defects is extensively used as a reliable solution, with high success rates. However, there are situations in which treatment with implants is ruled out or has very few possibilities of succeeding, due to shortcomings, medical treatments or the pathologies of the patients themselves.

The object of this invention is therefore to develop a procedure that personalises the surfaces of these implants, to correct the defects or treat the pathologies of each particular patient, in order to prevent rejection and increase the success rates of this treatment in the short, mid and long term, in addition to preventing disease in the tissues surrounding the implant.

It is known that oxygen, argon or helium plasma treatments exist for cleaning/activating the surfaces of certain materials, producing surfaces with radicals in which products react and thus facilitating adhesion.

Nonetheless, to the best of the applicant's knowledge, in reference to the prior art, no other procedure is known to exist that applies this plasma technology to implants and thus has similar technical characteristics to those of the present procedure which is claimed here.

Document US2014/004481A1 discloses a procedure for producing implants with a personalised surface according to the preamble of claim 1.

### EXPLANATION OF THE INVENTION

As mentioned earlier, the invention proposes a procedure for producing dental implants with a personalised surface by means of plasma technology, to obtain surfaces with no organic compound or oxide layers and completely activated, to which a liquid solution is then applied which, depending on the needs of each patient, may be osteoinductive, osteoconductive, antibiotic, probiotic or antibacterial.

This process will preferably be implemented in the dental clinic immediately before the implant placement procedure, but the initial step of cleaning the implant by applying plasma technology can be carried out in the implant production plant. In any event, personalisation by means of liquid solutions containing osteoinductive components or antibiotic, antibacterial or probiotic components will be carried out in the dental clinic and the selection thereof will be made in accordance with the patient's needs, to ensure the success of the implant osseointegration process.

The object of the invention is therefore focused on the process of personalising the implant surface by cleaning/activation with plasma technology, and the subsequent application of a specific liquid solution designed for each patient. This solution will already contain the osteoconductive, osteoinductive, antibiotic or probiotic components, separately or together, depending on the patient's needs.

As already indicated, the solution is applied to the implant after cleaning and activating the surface by means of plasma technology. After completing the cleaning/activation process, a vial containing the liquid solution is applied to the implant in its respective vial, and within a short while, the implant surface will be personalised for this patient.

The described procedure therefore essential involves the cleaning/activation of the implant surface by means of plasma technology, to make it chemically receptive and thus able to receive a solution that is specially designed for each patient, depending on their needs.

More specifically, the object of the invention is to clean/activate the implant surface by means of oxygen or argon plasma, to drag organic traces and contaminants, including the oxide layer that forms on the implant, activating the surface, creating sites with radicals and generating an extremely reactive surface to which the personalised solution is applied, and which, because of the reactive nature of the surface, will completely saturate the implant, giving it a personalised behaviour.

Through the personalisation of the implant surface, and depending on the biological characteristics of each patient, the success rate will increase substantially in the short, mid and long term, as the osseointegration process that occurs in dental implantology is accelerated and the BIC (bone implant contact) values between the bone and the implant are increased.

The cleaning/activation of surfaces by means of oxygen or argon plasma is known to be an extremely effective cleaning/activation method. Due to the effectiveness of the process, it is possible to obtain an extremely reactive surface that permits the application of the personalised solution.

### DETAILED EXPLANATION OF ONE METHOD OF IMPLEMENTATION

After planning the procedure, in other words, after studying the biology of the patient's jawbone, habits, medical history and analysing the patient's possible surgical trauma, based on the data obtained, the solution (which may be formed by a single osteoinductive, osteoconductive, antibacterial, antibiotic or probiotic compound, or a combination of any of them), is applied to the implant surface in order to increase the BIC (*bone implants contact*) values.

To ensure that the liquid penetrates all the micropores of the implant surface, before the implant procedure, the surface is cleaned/activated using plasma technology to obtain a surface with no innate titanium oxide layer in contact with air, making it highly reactive and allowing the corrective/enhancing liquid to penetrate the entire surface of the implant.

Normally, dental implants are supplied ready sterilised. The dental implant packaging consists of the implant in a vial, which in turn is inserted in a blister pack and hermetically sealed to maintain its sterility.

Thus, after obtaining the patient's data regarding the type of bone, biology, medical history and habits that could affect the correct osseointegration, the solution is selected with the most appropriate single or combined compound in each case, and it will therefore be an osteoinductive or an osteoconductive compound, an antibiotic or an antibacterial compound or a compound containing a combination of any of the above substances.

In particular, the types of solution used and their composition and quantity, depending on the different patient pathologies, will preferably be the following:

| | |
|---|---|
| - Probiotic fluid: | Promotes the development of non-pathogenic flora. |
| Quantity of fluid: | Vials containing between 2.5 cm³ and 5 cm³. |
| Composition: | Fluid comprised mainly of Lactobacillus or similar bacteria. |
| | |
| Patient pathologies: | |
| | - Osteopenia |
| | - Hyperparathyroidism. |
| | |
| - Antiobiotic fluid: | Promotes the inhibition of collagen and protein synthesis. |
| Quantity of fluid: | Vials containing between 2.5 cm³ and 5 cm³ |
| Composition: | Fluid comprised mainly of tetracycline, terramycin or similar drugs. |
| Patient pathologies: | - Decontaminants. |
| | - Prevention of periodontal and/or peri-implant disease recurrence. |
| | - Immunity problems. |
| | |
| - Osteoinductive fluid: | Promotes the formation of bone. |
| Quantity of fluid: | Vials containing between 2.5 cm³ and 5 cm³. |
| Composition: | Fluid of human origin (patient or donor), mostly made up of stem cells, platelet-derived growth factors or morphogenetic proteins. |
| Patient pathologies: | |
| | - Acute osteoporosis. |
| | - Chemotherapy or radiation therapy. |
| | - Bone mineralisation disorders. |
| - Osteoconductive fluid: | Promotes the maintenance of vital space for bone formation. |
| Quantity of fluid: | Vials containing between 2.5 cm³ and 5 cm³. |
| Composition: | Fluid consists mainly of hydroxyapatite and tricalcium phosphates. |
| Patient pathologies: | - Tooth extraction and immediately placement of implant. |
| | - Cyst cavities (bone defects). |
| | - Defects that complicate the healing of bone tissue. |

The implants to be placed in the patient's mouth undergo the plasma cleaning/activation process, which takes no more than 15 minutes, preferably at the time of surgery. For this purpose, the sealed blister must be opened and the vials must be extracted with the implants and placed in the plasma machine.

This plasma cleaning process has different phases. Firstly, the UV light generated in the plasma breaks up and lifts off most of the organic adhesives and surface contaminants. The particles dragged from the surface react chemically with the plasma oxygen ions, producing other molecules such as water and carbon dioxide, which are removed from the chamber by the vacuum system. In the plasmas generated for cleaning oxidable metals in which there is no oxygen, the particles removed from the surface are removed outside the chamber to prevent the re-depositing of material, leaving the surface ultra clean and ready for adherence.

Once the programmed cycle has ended, the vials are extracted with the implants inside them and the vial cap is removed. Without extracting the implants from the vial, the programmed sterile fluid is poured into the vial to reduce possible defects and/or enhance the patient's biological virtues and increase in the BIC values, thus improving the osseointegration of the implant in the patient's bone.

Then the implants are placed in the patient in the normal way.

Having described in sufficient detail the nature of this invention and the way in which it is put into practice, it is not considered necessary to make a more extensive description to allow an expert in the matter to understand its scope and the benefits it brings. It is stated that, within its essential design, it may be put into practice through other methods of implementation that differ in detail from what is indicated by way of example, with such methods being included within the scope of protection as defined by the appended claims.

## Claims

1. PROCEDURE FOR PRODUCING IMPLANTS WITH A PERSONALISED SURFACE, comprising the following steps:
- cleaning/activation of the dental implant surface by using oxygen or argon plasma for dragging organic traces and contaminants, including an oxide layer of the implant, in such a manner that sites with radicals are created and a reactive surface is generated; and
- application of a liquid solution designed specifically for each patient to the reactive surface of the dental implant, wherein the liquid solution designed for each patient is one selected from a osteoinductive, osteoconductive, antibiotic, probiotic or antibacterial solution **characterised in that** the cleaning/activation of the dental implant surface is carried out with the implant disposed inside a vial and the application of the liquid solution to the reactive surface is carried out into the vial containing the implant.

2. PROCEDURE FOR PRODUCING IMPLANTS WITH A PERSONALISED SURFACE, as set out in claim 1, **characterised in that** the liquid solution contains a single osteoinductive, osteoconductive, antibacterial, antibiotic or probiotic compound, or a combination of any of them.

3. PROCEDURE FOR PRODUCING IMPLANTS WITH A PERSONALISED SURFACE, as set out in claim 1 or 2, **characterised in that** the cleaning/activation of the implant surface by means of oxygen or argon plasma and application of the liquid solution are carried out in the dental clinic before the implant placement procedure.

4. PROCEDURE FOR PRODUCING IMPLANTS WITH A PERSONALISED SURFACE, as set out in claim 1 or 2, **characterised in that** the cleaning/activation of the implant surface using oxygen and argon plasma is carried out in the implant production plant and the liquid solution is applied in the dental clinic before the implant placement procedure.

## Patentansprüche

1. VERFAHREN ZUR HERSTELLUNG VON IMPLANTATEN MIT PERSONALISIERTER OBERFLÄCHE, umfassend die folgenden Schritte:
- Reinigen/Aktivieren der Oberfläche des Zahnimplantats unter Verwendung von Sauerstoff- oder Argonplasma zum Mitschleppen organischer Spuren und Verunreinigungen, einschließlich einer Oxidschicht des Implantats, derart, dass Stellen mit Radikalen entstehen und eine reaktive Oberfläche erzeugt wird; und
- Aufbringen einer flüssigen Lösung, die speziell für jeden Patienten gestaltet ist, auf die reaktive Oberfläche des Dentalimplantats, wobei die flüssige Lösung, die speziell für jeden Patienten gestaltet ist, eine, ausgewählt aus einer osteoinduktiven, osteokonduktiven, antibiotischen, probiotischen oder antibakteriellen Lösung ist, **dadurch gekennzeichnet, dass** die Reinigung/Aktivierung der Oberfläche des Dentalimplantats ausgeführt wird, während das Implantat in einem Fläschchen angeordnet ist, und das Aufbringen der flüssigen Lösung auf die reaktive Oberfläche in das Fläschchen, welches das Implantat enthält, ausgeführt wird.

2. VERFAHREN ZUR HERSTELLUNG VON IMPLANTATEN MIT PERSONALISIERTER OBERFLÄCHE nach Anspruch 1, **dadurch gekennzeichnet, dass** die flüssige Lösung eine einzelne osteoinduktive, osteokonduktive, antibakterielle, antibiotische oder probiotische Verbindung oder eine Kombination von ihnen enthält.

3. VERFAHREN ZUR HERSTELLUNG VON IMPLANTEN MIT PERSONALISIERTER OBERFLÄCHE nach Anspruch 1 oder 2, **dadurch gekennzeichnet, dass** die Reinigung/Aktivierung der Implantatoberfläche mittels Sauerstoff- oder Argonplasma und das Aufbringen der flüssigen Lösung in der Zahnklinik vor dem Implantatplatzierungsvorgang erfolgt.

4. VERFAHREN ZUR HERSTELLUNG VON IMPLANTEN MIT PERSONALISIERTER OBERFLÄCHE nach Anspruch 1 oder 2, **dadurch gekennzeichnet, dass** die Reinigung/Aktivierung der Implantatoberfläche unter Verwendung von Sauerstoff- und Argonplasma in der Implantatherstellungsanlage ausgeführt wird und die flüssige Lösung vor dem Implantatplatzierungsvorgang in der Zahnklinik aufgebracht wird.

## Revendications

1. PROCÉDÉ DE PRODUCTION D'IMPLANTS AVEC UNE SURFACE PERSONNALISÉE, comprenant les étapes suivantes :
- nettoyage/activation de la surface de l'implant dentaire en utilisant du plasma d'oxygène ou d'argon pour entraîner les traces organiques et les contaminants, comportant une couche d'oxyde de l'implant, de manière à créer des sites avec des radicaux et à générer une surface réactive ; et
- application d'une solution liquide conçue spécifiquement pour chaque patient sur la surface réactive de l'implant dentaire, dans lequel la solution liquide conçue pour chaque patient est une solution choisie parmi une solution ostéoinductrice, ostéoconductrice, antibiotique, probiotique ou antibactérienne **caractérisé en ce que** le nettoyage/l'activation de la surface de l'implant dentaire est effectué avec l'implant disposé à l'intérieur d'un flacon et l'application de la solution liquide sur la surface réactive est effectuée dans le flacon contenant l'implant.

2. PROCÉDÉ DE PRODUCTION D'IMPLANTS AVEC UNE SURFACE PERSONNALISÉE, selon la revendication 1, **caractérisé en ce que** la solution liquide contient un seul composé ostéoinducteur, ostéoconducteur, antibactérien, antibiotique ou probiotique, ou une combinaison de l'un d'entre eux.

3. PROCÉDÉ DE PRODUCTION D'IMPLANTS AVEC UNE SURFACE PERSONNALISÉE, selon la revendication 1 ou 2, **caractérisé en ce que** le nettoyage/l'activation de la surface de l'implant au moyen de plasma d'oxygène ou d'argon et l'application de la solution liquide sont effectués dans la clinique dentaire avant le procédé de pose de l'implant.

4. PROCÉDÉ DE PRODUCTION D'IMPLANTS AVEC UNE SURFACE PERSONNALISÉE, selon la revendication 1 ou 2, **caractérisé en ce que** le nettoyage/l'activation de la surface de l'implant en utilisant du plasma d'oxygène et d'argon est effectué dans l'usine de production d'implants et la solution liquide est appliquée dans la clinique dentaire avant le procédé de pose de l'implant.
